Europäisches Patentamt

European Patent Office

Office européen des brevets

(11) Veröffentlichungsnummer: **0 358 853**
**A2**

## (12) EUROPÄISCHE PATENTANMELDUNG

(21) Anmeldenummer: **89110450.7**

(22) Anmeldetag: **09.06.89**

(51) Int. Cl.5: **C01B 15/037**

(30) Priorität: **18.06.88 DE 3820726**

(43) Veröffentlichungstag der Anmeldung:
**21.03.90 Patentblatt 90/12**

(84) Benannte Vertragsstaaten:
**AT BE CH DE ES FR GB GR IT LI NL SE**

(71) Anmelder: **Henkel Kommanditgesellschaft auf Aktien**
**Postfach 1100 Henkelstrasse 67**
**D-4000 Düsseldorf 1(DE)**

(72) Erfinder: **Jacobs, Jochen, Dr.**
**Am Acker 20**
**D-5600 Wuppertal 1(DE)**
Erfinder: **Matzik, Iduna, Dr.**
**Auf der Gathe 20**
**D-4300 Essen 15(DE)**
Erfinder: **Höffkes, Horst, Dr.**
**Carlo-Schmid-Strasse 113**
**D-4000 Düsseldorf-Hellerhof(DE)**
Erfinder: **Seidel, Kurt**
**Nosthoffenstrasse 59**
**D-4000 Düsseldorf 13(DE)**

(54) **Stabilisierende teilchenförmige Wasserstoffperoxid-Addukte und deren Verwendung.**

(57) Teilchenförmige Wasserstoffperoxid-Additionsverbindungen werden mit wasserlöslichen, freie Carboxylgruppen tragenden Homo- oder Copolymerisaten, bevorzugt der Acrylsäure oder Methacrylsäure, in der Säureform umhüllt. Dadurch wird die Lagerstabilität erhöht ohne daß Probleme bei der Wasserstoffperoxid-Entwicklung bei Zutritt von Wasser auftreten. Bevorzugt wird eine wäßrige Lösung des Polymerisats in einer Wirbelschicht aufgesprüht und das Wasser durch Trocknung entfernt.

EP 0 358 853 A2

EP 0 358 853 A2

## Stabilisierte teilchenförmige Wasserstoffperoxid-Addukte und deren Verwendung

Die Erfindung betrifft stabilisierte, teilchenförmige Wasserstoffperoxid-Additionsverbindungen, ein Verfahren zu deren Herstellung durch Umhüllen mit wasserlöslichen, carboxylgruppenhaltigen Polymerisaten in der Säureform sowie deren Verwendung als Oxidationsmittelkomponente in pulverförmigen Oxidationshaarfärbemitteln.

Teilchenförmige Wasserstoffperoxid-Addukte werden für zahlreiche Bleich- und Oxidationsvorgänge verwendet, besonders in pulverförmigen Zubereitungen, in welchen sie oft gemeinsam mit anderen Komponenten, z. B. mit Tensiden, Farbstoffen, optischen Aufhellern, Duftstoffen, antimikrobiellen und anderen oxidationsempfindlichen Stoffen enthalten sind. Ein besonderes Problem stellt dabei die Stabilität des Wasserstoffperoxid-Adduktes dar, welches sich -besonders unter dem Einfluß von Feuchtigkeit - leicht unter Sauerstoffentwicklung zersetzt und dabei die anderen oxidationsempfindlichen Komponenten der Zubereitungen schädigt.

Es hat daher nicht an Vorschlägen gefehlt, teilchenförmige Wasserstoffperoxid-Addukte durch Zusätze, Granulierhilfsmittel oder auch durch Umhüllung (das sogenannte Coating) mit den verschiedenen Stoffen vor vorzeitiger Zersetzung zu bewahren.

Aus DE 24 02 392 C2 ist z. B. ein Verfahren bekannt, durch Umhüllung mit einem Mischpolymerisat aus Vinylideinchlorid und einem anderen polymerisierbaren Monomeren, z. B. aus Vinylchlorid, Acrylnitril oder einem Acrylsäure- oder Methacrylester zu stabilisieren.

Solche, mit wasserunlöslichen Polymeren umhüllten Partikel von Wasserstoffperoxid-Addukten haben, besonders bei der Verwendung als Komponente in pulverförmigen Haarfärbemitteln den Nachteil, daß sie, beim Ansetzen des Färbemittels mit Wasser, nicht rasch genug das Wasserstoffperoxid frei geben und daher eine verzögerte, unbefriedigende Entwicklung des Oxidationsfarbstoffs verursachen, was zu Verschiebungen der Nuance und zu ungleichmäßiger Haarfärbung führt.

Aus anderen Druckschriften, z. B. Norda Briefs No. 445, December 1972, war es bekannt, bestimmte Wasserstoffperoxid-Addukte, z. B. Trinatriumcitrat-diperhydrat durch "Coating" mit Fettsäure, Wachs, Paraffin, Polyvinylalkohol oder Celluloseestern zu stabilisieren. Die auf diese Weise erhaltenen Addukte zeigen zwar eine etwas verbesserte Lagerstabilität, die Wirkung der nicht wasserlöslichen Hüllmaterialien ist aber aus den oben beschriebenen Gründen für die Verwendung in Oxidationshaarfärbepulvern ungeeignet, bei den wasserlöslichen, polymeren Hüllmaterialien, z. B. beim Polyvinylalkohol besteht das Problem der zu geringen Stabilität gegenüber Feuchtigkeit, insbesondere wenn basische Komponenten in der Zubereitung enthalten sind.

In DE 36 36 904 wird ein Verfahren zur Umhüllung von Persäuregranulaten beschrieben. Die dort beschriebenen Peroxycarbonsäuren sind aber nicht als Oxidationsmittelkomponente für Oxidationshaar färbemittel geeignet, da sie den Oxidationsfarbstoff zerstören.

In DE 34 34 468 wird ein Verfahren zur Herstellung pulverförmiger, fließfähiger Wasserstoffperoxid-Addukte beschrieben, wobei die pulverförmigen Wasserstoffperoxid-Anlagerungsverbindungen mit einem Polyalkylenglycol, z. B. mit Polyethylenglycol mit einem mittleren Molekulargewicht von 200 - 400 in einer Menge von 0,5 bis 5,0 Gewichtsprozent trocken vermischt werden. Auch dabei kommt es zu einer Umhüllung der Teilchen des Addukts, die Produkte neigen jedoch bei Zutritt von Luftfeuchtigkeit immer noch zur Verklebung und zur Instabilität, wenn sie in Pulverzubereitungen mit alkalischen Komponenten eingesetzt werden.

Es bestand daher die Aufgabe, teilchenförmige Wasserstoffperoxid-Addukte gegen Zersetzung und gegen den Angriff von Luftfeuchtigkeit wirksamer zu stabilisieren und solche stabilisierten teilchenförmigen Addukte zu finden, die sich besser als die bekannten Produkte als Komponenten zur Herstellung von Oxidationshaarfärbemitteln in Pulverform eignen.

Gegenstand der Erfindung sind stabilisierte, teilchenförmige Wasserstoffperoxid-Additionsverbindungen, die mit wasserlöslichen Polymerisaten umhüllt sind, und deren Hülle aus einem wasserlöslichen, freie Carboxylgruppen tragenden Homo- oder Copolymerisat besteht. Als freie Carboxylgruppen werden solche bezeichnet, die in der Säureform (-COOH), nicht in neutralisierter Salzform, vorliegen.

Als Wasserstoffperoxid-Additionsverbindungen oder "Peroxid-Addukte" eignen sich die bekannten festen Addukte von Wasserstoffperoxid an anorganische oder organische Moleküle wie z. B. Natrium perborat-monohydrat $(NaBO_2 \cdot H_2O_2)$, Natriumperborat-tetrahydrat $(NaBO_2 \cdot H_2O_2 \cdot 3H_2O)$, Perborax $(Na_2B_4O_7 \cdot 4H_2O_2)$, Peroxypyrophosphate, Citratperhydrat (z. B. gemäß DE 19 20 831) Amino-1,3,5-triazinperhydrat (Melamin-Perhydrat, gemäß DE 11 03 934) oder Harnstoffperhydrat (Percarbamid, gemäß US 3 629 331). Besonders geeignet sind solche teilchenförmigen Addukte, die eine Korngrößenverteilung aufweisen, bei der mehr als 90 % der Partikel einen Durchmesser von 0,1 bis 1,0 mm und weniger als 1 %

2

einen Durchmesser von mehr als 1,0 mm haben.

Als wasserlösliche, freie Carboxylgruppen tragende Polymerisate eignen sich alle Homo- und Copolymerisate von ungesättigten Carbonsäuren, z. B. von Acrylsäure, Methacrylsäure, Crotonsäure, alpha-Hydroxyacrylsäure, Maleinsäure, Alkylessigsäure, 2-Alkyloxypropionsäure, 2-Vinylpropionsäure oder Vinylessigsäure.

Als Comonomere können z. B. Styrol, Acryl- oder Methacrylsäureester, Acrylamid, Methacrylamid, Diester der Malein- und Fumarsäure Vinylester und Vinylether eingesetzt werden, allerdings sollten nicht mehr als bis zu 20 Mol-% dieser nicht wasserlöslichen Comonomeren eingesetzt werden.

Bevorzugt geeignet sind die Homo- und Copolymerisate der Acrylsäure oder Methacrylsäure. In einer bevorzugten Ausführungsform besteht das Hüllmaterial aus einer Polyacrylsäure mit einer spezifischen Viskosität im Bereich von 0,07 bis 0,9, bevorzugt von 0,10 bis 0,20. Als spezifische Viskosität (v spez.) wird dabei der Quotient $(v - v_o)/v_o$ verstanden, wobei v die Viskosität einer Lösung des Natriumsalzes und $v_o$ die Viskosität des Lösungsmittels ist. Im vorliegenden Fall wurde jeweils die Viskosität einer 0,7 %igen Lösung des Na-Salzes (Gew.-%) in einer 2-n-Natronlauge gemessen.

Das Hüllmaterial macht bevorzugt 2 - 20 Gew.-% des umhüllten Wasserstoffperoxid-Anlagerungsverbindung aus.

Die Herstellung der erfindungsgemäß stabilisierten, teilchenförmigen Wasserstoffperoxid-Additionsverbindungen kann im einfachsten Fall dadurch erfolgen, daß man eine wäßrige Lösung des freie Carboxylgruppen tragenden Homo- oder Copolymerisats auf die teilchenförmigen Wasserstoffperoxid-Additionsverbindungen aufsprüht und diese gleichzeitig und/oder anschließend trocknet. Bei diesem Vorgang kommt es zu einer Umhüllung der Teilchen der Wasserstoffperoxid-Addukte durch das Polymerisat.

Zur Durchführung dieses Umhüllungsverfahrens eignen sich zahlreiche Geräte, die zur Trocknung pulverförmiger Feststoffe geeignet sind.

Um bei diesem Coating-Verfahren eine Verklebung der Partikel und eine Kornvergröberung durch Granulationsvorgänge zu verhindern, ist es vorteilhaft, die Trocknung unter ständiger Bewegung der Partikel mit einem erwärmten Luftstrom durchzuführen.

Eine bevorzugte Ausführungsform des erfindungsgemäßen Verfahrens zeichnet sich dadurch aus, daß das Aufsprühen der wäßrigen Lösung des Homo- oder Copolymerisats und das Trocknen der teilchenförmigen Wasserstoffperoxid-Additionsverbindung in einer Wirbelschicht unter Zuführung eines erwärmten Luft- oder Inertgasstromes durchgeführt wird. Als Inertgas kann z. B. Stickstoff verwendet werden. Der Luft- oder Inertgasstrom wird in dem speziell für dieses Ver fahren geeigneten "Wirbelschicht-Coater" durch einen Siebboden eingeleitet, auf welchem sich das teilchenförmige Material befindet. Dieses wird durch den Luft- oder Inertgasstrom in eine Wirbelschicht überführt, in der die einzelnen Partikel in ständiger Bewegung sind. Die Temperatur des Luft- oder Inertgasstroms wird dabei so hoch eingestellt, daß das fluidisierte teilchenförmige Material auf ca. 35 - 50 °C erwärmt wird.

Nachdem die Trocknung des umhüllten, teilchenförmigen Wasserstoffperoxids abgeschlossen ist, wird es durch Zuführung eines Kaltluftstroms auf eine Temperatur unterhalb 35 °C abgekühlt.

In einer bevorzugten Ausführung des erfindungsgemäßen Verfahrens wird das Homo- oder Copolymerisat in einer Menge von 2 - 20 Gewichtsteilen in Form einer 5 - 30 Gew.-%igen wäßrigen Lösung auf 80 - 98 Gewichtsteile der Wasserstoffperoxid-Additionsverbindung aufgesprüht.

Die erfindungsgemäß umhüllten und auf diese Weise stabilisierten teilchenförmigen Wasserstoffperoxid-Additionsverbindungen weisen eine erhöhte Lagerstabilität, auch bei erhöhten Temperaturen, etwa bis + 50 °C auf. Sie eignen sich als Oxidationsmittel für pulverförmige Zubereitungen für zahlreiche Bleich- und Oxidationsverfahren.

Besonders eignen sich die erfindungsgemäßen teilchenförmigen Wasserstoffperoxid-Additionsverbindungen als Oxidationsmittelkomponente in pulverförmigen Oxidationshaarfärbemitteln.

Oxidationshaarfärbepulver, wie sie z. B. aus DE 11 41 749 oder aus DE 11 77 775 bekannt waren, bestehen aus einem pulverförmigen Gemisch von Oxidationsfarbstoffenvorprodukten, pulverförmigen, wasserlöslichen Verdickungsmitteln wowie ggf. pulverförmigen Streckmitteln, Tensiden, Stabilisatoren und einer teilchenförmigen Wasserstoffperoxid-Additionsverbindung als Oxidationsmittelkomponente.

Die erfindungsgemäß stabilisierten teilchenförmigen Wasserstoffperoxid-Additionsverbindungen weisen in solchen Zusammensetzungen folgende technische Vorteile auf: Die Viskosität der aus den Oxidationshaarfärbepulvern durch Ansetzen in Wasser erhaltenen Färbezubereitungen bleibt - auch nach längerer Lagerung des Pulvers -unverändert, da der Angriff der Oxidationsmittelkomponente auf das polymere Verdickungsmittel wirksam verhindert wird. Der Gehalt an aktivem Sauerstoff bleibt über längere Lagerzeiten konstant. Die durch Ansetzen der Oxidationshaarfärbepulver in Wasser erhaltenen Färbezubereitungen weisen eine größere Glätte und Gleichmäßigkeit auf, da der Quellprozeß dar pulverförmigen, wasserlöslichen Verdickungsmittel günstig beeinflußt wird.

Die mit den erfindungsgemäß stabilisierten, teilchenförmigen Wasserstoffperoxid-Additionsverbindungen hergestellten pulverförmigen Oxidationshaarfärbemittel weisen bevorzugt folgende Zusammensetzung auf:

| 0,04 - 0,4 | Mol/100g | an Oxidationsfarbstoffvorprodukten, |
|---|---|---|
| 1 - 20 | Gew.-% | eines wasserfreien Zeolith-Trocknungsmittels, |
| 10 - 30 | Gew.-% | eines wasserlöslichen polymeren Verdickungsmittels, |
| 10 - 40 | Gew.-% | einer erfindungsgemäß stabilisierten, teilchenförmigen Wasserstoffperoxid-Additionsverbindung, |
| 2 - 20 | Gew.-% | eines pulverförmigen, wasserlöslichen Tensids. |

Als Oxidationsfarbstoffvorprodukte werden Entwicklersubstanzen und gegebenenfalls auch Kupplersubstanzen eingesetzt.

Die Entwicklerkomponenten bilden unter dem Einfluß des Oxidationsmittels oder von Luftsauerstoff untereinander oder unter Kupplung mit einer oder mehreren Kupplerkomponenten die eigentlichen Farbstoffe aus.

Als Entwicklersubstanzen werden üblicherweise primäre, aromatische Amine mit einer weiteren in Para- oder Orthoposition befindlichen freien oder substituierten Hydroxy- oder Aminogruppe oder deren Salze, ferner Diaminopyridinderivate, heterocyclische Hydrazonderivate, 4-Aminopyrazolonderivate und 2,4,5,6-Tetraaminopyridin und dessen Derivate eingesetzt. Besonders wichtige Entwicklersubstanzen sind p-Phenylendiamin, p-Toluylendiamin, p-Aminophenol und 2,4,5,6-Tetraaminopyrimidin.

Zur Modifikation der Nuancen und zur Erhöhung der Brillanz und Echtheit werden Kupplerverbindungen eingesetzt. Als Kupplersubstanzen eignen sich z. B. m-Phenylendiamine, m-Aminophenole oder deren Salze, m-Dihydroxybenzol, Naphtol-1, 1,5- und 2,7-Dihydroxynaphtalin, Hydroxy- und Aminopyridine, Hydroxychinoline und Aminopyrazolone. Besonders wichtige Kupplersubstanzen sind m-Phenylendiamin, 3-Aminophenol und m-Dihydroxybenzol und deren Derivate. Entwickler- und Kupplersubstanzen werden im allgemeinen in äquimolaren Mengen eingesetzt, ein gewisser Überschuß einzelner Oxidationsfarbstoffvorprodukte ist jedoch nicht nachteilig, so daß Entwicklersubstanzen und Kupplersubstanzen in einem Molverhältnis von 1 : 0,5 bis 1 : 2 eingesetzt werden können. In den Oxidationshaarfärbepulvern werden die Entwicklerkomponenten bevorzugt in einer Menge von 0,02 bis 0,2 Mol/100g und die Kupplerkomponenten bevorzugt in einer Menge von 0,01 bis 0,2 Mol/100 g eingesetzt.

Das Zeolith-Trocknungsmittel ist vorzugsweise ein natürliches, insbesondere ein synthetisches, ganz oder weitgehend wasserfreies kristalline Alkali-Aluminium-Silikat mit Zeolith-Struktur, das im allgemeinen nicht mehr als 3, vorzugsweise nicht mehr als 1,5 % Wasser enthält und infolgedessen ein hohes Feuchtigkeits-Adsorptionsvermögen besitzt. Besonders geeignet sind die synthetischen Zeolithe des Typs A und/oder X in Pulverform. Solche Produkte sind im Handel erhältlich, z. B. Baylith[R]-T-Pulver (Bayer AG), ein Zeolith vom Typ NaA mit einem Schüttgewicht von 400 bis 450 g/dm$^3$; oder die Produkte "Molekularsiebe UETIKON Puder" der Firma Chem. Fabr. UETIKON (CH), von denen ebenfalls vorzugsweise die Natriumformen der Zeolithe A und X verwendet werden. Brauchbar sind aber auch die genannten Zeolithe in gekörnter Form, d. h. als Agglomerate von ca. 1 bis 2 mm Durchmesser, die man als bindemittelhaltige oder -freie Produkte verwenden kann. Erfindungsgemäß brauchbar, wenn auch mit schwächerer Stabilisatorwirkung, sind auch getrocknete amorphe Natriumaluminiumsilikate als Pulver oder in gekörnter Form, die auch bei einem höheren Wassergehalt als 3 % als Trocknungsmittel wirken und hier ebenfalls unter dem Begriff Zeolith-Trocknungsmittel verstanden werden sollen.

Nach dem Anrühren mit Wasser sollen die Oxidationshaarfärbepulver eine cremige oder gelförmige Färbezubereitung ergeben. Dies er fordert einen Gehalt an einem wasserlöslichen Verdickungsmittel. Als solche eignen sich natürliche und synthetische Polymere in pulverförmiger oder feinteiliger, rieselfähiger Form, die sich in Wasser unter starker Viskositätserhöhung auflösen. Beispiele für solche Polymere sind wasserlösliche Derivate von Cellulose, z. B. Carboxymethylcellulose, Methylcellulose, Hydroxyethylcellulose, Alginate, Stärkederivate und andere, wasserlösliche Polysaccharide wie z. B. Guar-Gum, wasserlösliche Guar-Derivate und Xanthan-Gum. Auch synthetische Polymere wie z. B. Polyacrylamide und Copolymerisate von Acrylsäure, Methacrylsäure oder Crotonsäure mit Vinylverbindungen können verwendet werden.

Bevorzugt eignet sich als wasserlösliches Verdickungsmittel Xanthan-Gum, ein Biopolymer, der durch Fermentation von Kohlenhydraten mit einem Mikroorganismus des Xanthomonas-Typs erzeugt wird und dessen Makromolekül sich hauptsächlich aus Glucose-, Mannose-und Glucuronsäure-Einheiten zusammensetzt. Dieser Xanthan-Gum wird bevorzugt in einer Menge von 10 bis 30 Gew.-% eingesetzt.

Zur Erleichterung des Auflösevorgangs und zur Erzeugung einer gleichmäßigen Haarfärbung enthalten die Oxidationshaarfärbepulver ein oder mehrere feinteilige bzw. pulverförmige, wasserlösliche Tenside.

Besonders geeignet sind die Alkalisalze von Schwefelsäurehalbestern der linearen oder wenig verzweigten Fettalkohole mit 10 -16 C-Atomen, z. B. Natriumlaurylsulfat. Weitere geeignete pulverförmige anionische Tenside sind z. B. die Alkalisalze von linearen und verzweigtkettigen Alkylbenzolsulfonaten mit 6 - 16 C-Atomen in der Alkylgruppe, Alken- und Hydroxyalkansulfonate, wie sie durch Sulfonierung von alpha-Olefinen mit 10 - 18 C-Atomen erhalten werden, Sulfobernsteinsäuremonoalkylester-Alkalisalze mit 8 - 18 C-Atomen in der Alkylgruppe, Sulfobernsteinsäuredialkylester-Alkalisalze mit 6 - 10 C-Atomen in der Alkylgruppe, Mono- und Dialkylnaphthalinsulfonat-Alkalisalze mit 1 - 8 C-Atomen in der Alkylgruppe, Alkylpolyglycolethercarboxylate mit 8 - 18 C-Atomen in der Alkylgruppe und 2 - 6 Glycolethergruppen im Molekül, Acylsarkosine, Acyltauride und Acylisethionate mit 8 - 18 C-Atomen in der Acylgruppe und alpha-Sulfofettsäuremethylester-Alkalisalze von Fettsäuren mit 8 - 18 C-Atomen.

Geeignet sind auch feste, feinkörnige Zubereitungen aus oberflächenaktiven Stoffen, die sich in reiner Form nicht in freifließende Pulverform bringen lassen, und inerten Hilfs- und Trägerstoffen, die eine nicht klebrige, feinkörnige, freifließende Zubereitung solcher, in reiner Form pastösen, niedrigschmelzenden oder klebrigen Tenside ermöglichen. Solche geeigneten Hilfs- und Trägerstoffe sind bevorzugt gut wasserlöslich. Wasserlösliche Hilfs- und Trägerstoffe sind z. B. anorganische wasserlösliche Salze wie z. B. Natriumsulfat, Natriumchlorid, Natriumcarbonat, Natriumhydrogencarbonat, Natriumphosphat, gegebenenfalls auch die entsprechenden Kalium- oder Ammoniumsalze. Auch andere, nicht salzartige wasserlösliche Trägerstoffe wie z. B. Harnstoff oder wasserlösliche Mono- und Disaccharide können verwendet werden.

Die Oxidationshaarfärbepulver können darüber hinaus weitere Hilfsmittel in untergeordneten Mengen, etwa von 0,5 - 5 Gew.-%, enthalten, z. B. direktziehende Farbstoffe, Duftstoffe, Komplexbildner, Natriumsulfit, feinteilige Kieselsäure oder Pigmente. Auch haarkosmetische Hilfsmittel zur Verbesserung der kosmetischen Eigenschaften des Haares können enthalten sein. Unter diesen Hilfsmitteln kommt den kationischen, wasserlöslichen Polymeren eine besondere Bedeutung zu, da sie dem Haar eine gute Kämmbarkeit und Fülle geben und einen besseren Halt der Frisur bewirken.

Weitere haarkosmetische Hilfsmittel, die mit Vorteil zur Verbesserung der Kämmbarkeit und zur Verringerung der statischen Aufladung des Haares zugesetzt werden, sind kationische oberflächenaktive Substanzen vom Typ der quartären Ammoniumverbindungen. Dies sind bevorzugt Verbindungen, die ein quartäre Ammoniumgruppe in Form des Chlorids, Bromids, Sulfats, Phosphats, Methosulfats oder Ethosulfats tragen, die mit ein oder zwei langkettigen, bevorzugt linearen Alkyl-, Hydroxyalkyl. oder Alkyl-(poly)-oxyethylgruppen mit 10 bis 22 C-Atomen in der Alkyl- oder Hydroxyalkylgruppe und einer oder zwei Alkyl-, Hydroxyalkyl- oder Polyhydroxyalkylgruppen mit 1 bis 4 C-Atomen in der Alkylgruppe und ggf. einer Benzylgruppe substituiert sind. Geeignet sind auch Alkylpyridinium-Salze und Alkyl-Imidazoliniumsalze mit 10 bis 22 C-Atomen in der Alkylgruppe. Solche quartären Ammoniumverbindungen werden bevorzugt in einer Menge von 0,1 bis 10 Gew.-% den erfindungsgemäßen Oxidationshaarfärbepulvern zugesetzt. Beispiele für geeignete quartäre Ammoniumverbindungen sind Cetyltrimethylammoniumchlorid, Lauryldimethylbenzyl-ammoniumchlorid, Stearyl-tris-(polyoxyethyl)-ammonium-phosphat, Cetylpyridiniumchlorid, Distearyl-dimethylammoniumchlorid oder 2-Hydroxyhexadecyl-2-hydroxyethyl-dimethyl-ammoniumchlorid.

Die Herstellung der Oxidationshaarfärbemittelpulver erfolgt durch Vermischen der Komponenten.

Die Anwendung der Oxidationshaarfärbemittel erfolgt in einfacher Weise dadurch, daß man 1 Gewichtsteil des Pulvers mit 5 bis 15 Gewichtsteilen Wasser vermischt. Dabei bildet sich eine cremige bis gelförmige Färbezubereitung mit einem pH-Wert, der in einem Bereich von ca. 6 bis 9 liegen kann. Diese läßt sich, z. B. mit einem Pinsel, auf das Haar auftragen. Nach einer Einwirkzeit von ca. 5 bis 30 Minuten spült man überschüssiges Färbemittel aus dem Haar aus.

Die folgenden Beispiel sollten den Gegenstand der Erfindung näher erläutern, ohne ihn hierauf zu beschränken.

Beispiele

1. Umhüllen von Natriumperborat-Monohydrat ($NaBO_2 \cdot H_2O_2$) mit Polyacrylsäure

5

1.1 950 g Natriumperborat-Monohydrat (1) wurden in einem Wirbelschicht-Coater des Typs "Uniglatt" (Hersteller: GLATT, ) vorgelegt. Das Pulver wurde durch einen auf ca. 100 °C vorgeheizten Luftstrom fluidisiert, dessen Strömungsgeschwindigkeit am Siebboden des Gerätes ca. 1 - 1,2 m/s betrug. Auf das fluidisierte Material wurden 263 g einer 20 Gew.-%igen wäßrigen Lösung einee Polyacrylsäure (2) über eine mit 3 bar Druckluft betriebene 1,2 mm-Zweistoffdüse aufgebracht. Bei einer Sprührate von 18 g/min stellte sich im Wirbelbett eine konstante Temperatur von ca. 47 °C ein. Nach Abschluß des Aufdüsens wurde das Produkt (3) durch Wirbelung mit Kaltluft auf eine Temperatur unterhalb 35 °C abgekühlt.

Es wurden folgende Rohstoffqualitäten eingesetzt

(1) Natriumperborat-Monohydrat ($NaBO_2 \cdot H_2O_2$)

| Aktivsauerstoffgehalt: | | 15,3 Gew.-% |
|---|---|---|
| Schüttgewicht: | | 590 kgm$^3$ |
| Korngrößenverteilung: | über 0,8 mm | 0,1 Gew.-% |
| | über 0,1 mm | 99,9 Gew.-% |

Aussehen: weiße kristalline, frei fließende Substanz

| (2) Polyacrylsäure | |
|---|---|
| Aussehen: feines weißes Pulver spezifische Viskosität: (0,7 Gew.-% in 2n NaOH-Lösung) | 0,15 |
| pH-Wert (20 Gew.-% in Wasser): | 2,0 |

Das gecoatete Natriumperborat-Monohydrat (3) zeigte folgende Kenndaten:

| Schüttgewicht: | | 610 | g/l |
|---|---|---|---|
| Korngrößen-Verteilung: | über 0,8 mm | 0,7 | Gew.-% |
| Sieb-Analyse | über 0,1 mm | 99,8 | Gew.-% |
| Aktivsauerstoff-Gehalt: | | 14,5 | Gew.-% |
| Gewichtsanteil Hüllmaterial: | | 5,2 | Gew.-% |

1.2 In einem zweiten Ansatz wurden unter sonst gleichen Bedingungen wie in 1.1 auf 890 g Natriumperboratmonohydrat (1) ca. 556 g einer 20 Gew.-%igen wäßrigen Lösung der Polyacrylsäure (2) aufgebracht. Das dabei erhaltene gecoatete Produkt (4) zeigte folgende Kenndaten:

| Schüttgewicht: | | 630 | g/l |
|---|---|---|---|
| Korngrößen-Verteilung: | über 0,8 mm: | 0,6 | Gew.-% |
| (Siebanalyse) | über 0,1 mm: | 99,9 | Gew.-% |
| Aktivsauerstoff-Gehalt: | | 13,6 | Gew.-% |
| Gewichtsanteil Hüllmaterial: | | 11,1 | Gew.-% |

2. Herstellung eines Oxidationshaarfärbemittel-Pulvers

Durch Mischen der Komponenten wurde folgende Zusammensetzung hergestellt:

|                                                           | Gew.-% |
|-----------------------------------------------------------|--------|
| p-Phenylendiamin                                          | 21,0   |
| p-Aminophenol                                             | 1,0    |
| Na-Aluminium-Silikat (Baylith L)[1)]                      | 8,0    |
| Na-Alginat                                                | 17,0   |
| Hydroxyethylcellulose                                     | 9,0    |
| Na-Perborat-monohydrat aus Beispiel 1.1                   | 30,0   |
| Na-Laurylsulfat                                           | 10,0   |
| Kieselsäure (Aerosil 200)                                 | 1,0    |
| Kationisches Cellulosederivat (Polymer IR 400, Union Carbide) | 1,0 |
| $Na_2SO_3$                                                | 2,0    |

Das Produkt ergibt nach Zugabe von 9 Gewichtsteilen Wasser zu 1 Gewichtsteil der Zusammensetzung eine gebrauchsfertige Färbezubereitung zur Erzeugung einer tiefbraunen Haarfärbung.

Die Zusammensetzung zeigte nach 24 Stunden Lagerzeit bei 35 °C, 45 °C, 50 °C und 60 °C keine Viskositätsänderung der damit hergestellten Färbezubereitungen.

Werden sonst gleich zusammengesetzten Oxidationshaarfärbemittel-Pulver, die ein nicht erfindungsgemäß stabilisiertes Na-Perborat-Monohydrat enthalten, 24 Stunden bei erhöhter Temperatur gelagert, so zeigen die damit hergestellten Färbezubereitungen stark erhöhte Viskositätswerte, z. B. nach 24 Stunden Lagerung bei 50 °C eine Viskositätserhöhung um 150 %.

**Ansprüche**

1. Stabilisierte, teilchenförmige Wasserstoffperoxid-Additionsverbindungen, die mit wasserlöslichen Polymerisaten umhüllt sind, dadurch gekennzeichnet, daß die Hülle aus einem wasserlöslichen, freie Carboxylgruppen tragenden Homo- oder Copolymerisat besteht.

2. Stabilisierte, teilchenförmige Wasserstoffperoxid-Additionsverbindungen nach Anspruch 1, dadurch gekennzeichnet, daß das Hüllmaterial aus einem wasserlöslichen Homo- oder Copolymerisat der Acrylsäure- oder Methacrylsäure in der Säureform besteht.

3. Stabilisierte, teilchenförmige Wasserstoffperoxid-Additionsverbindungen nach Anspruch 1 oder 2, dadurch gekennzeichnet, daß die Wasserstoffperoxid-Additionsverbindung aus Natriumperboratmonohydrat besteht.

4. Stabilisierte, teilchenförmige Wasserstoffperoxid-Additionsverbindungen nach Anspruch 1 - 3, dadurch gekennzeichnet, daß das Hüllmaterial aus einer Polyacrylsäure mit einer spezifischen Viskosität im Bereich von 0,1 bis 0,2 in einer Menge von 2 - 20 Gew.-% des gesamten Produktes besteht.

5. Verfahren zur Herstellung stabilisierter, umhüllter teilchenförmiger Wasserstoffperoxid-Additionsverbindungen nach Anspruch 1 - 4, dadurch gekennzeichnet, daß man eine wäßrige Lösung des freie Carboxylgruppen tragenden Homo- oder Copolymerisats auf die teilchenförmige Wasserstoffperoxid-Additionsverbindungen aufsprüht und diese gleichzeitig und/oder anschließend trocknet.

6. Verfahren nach Anspruch 5, dadurch gekennzeichnet, daß das Aufsprühen der wäßrigen Lösung des Homo- oder Copolymerisats und das Trocknen der teilchenförmigen Wasserstoffperoxids-Additionsverbindung in einer Wirbelschicht unter Zuführung eines erwärmten Luft- oder Inertgasstromes durchgeführt wird.

7. Verfahren nach Anspruch 5 oder 6, dadurch gekennzeichnet, daß man 2 - 20 Gewichtsteile des Homo- oder Copolymerisats in Form einer 5 - 30 Gew.-%igen wäßrigen Lösung auf 80 - 98 Gewichtsteile der Wasserstoffperoxid-Additionsverbindung aufsprüht.

8. Verwendung von stabilisierten, teilchenförmigen Wasserstoffperoxid-Additionsverbindungen gemäß Anspruch 1 - 4 als Oxidationsmittelkomponente in pulverförmigen Oxidationshaarfärbezubereitungen.